# EUROPEAN PATENT APPLICATION

(11) **EP 4 101 374 A1**
(43) Date of publication of application: **14.12.2022**
(21) Application number: 22710946.9
(22) Date of filing: 01.03.2022
(51) Int. Cl.: A61B 5/024

(54) **WEARABLE DEVICE, AND HEART RATE TRACKING METHOD THEREFOR AND HEART RATE TRACKING APPARATUS THEREOF**

(30) Priority: 26.04.2021 CN 202110455923
(71) Applicant: Anhui Huami Healthcare Co., Ltd., Hefei, Anhui 243000 (CN)
(72) Inventor: ZHAO, Mingxi, Hefei, Anhui 243000 (CN)
(74) Representative: Papa, Elisabetta
(86) International application number: PCT/CN2022/078681
(87) International publication number: WO 2022/227843

(57) **Abstract**

The present disclosure provides a wearable device, as well as a heart rate tracking method and a heart rate tracking apparatus thereof. The method includes obtaining a photo pethysmo graphic (PPG) signal of the wearable device within a preset time, obtaining a frequency spectrum corresponding to the PPG signal by a short-time Fourier transform of the PPG signal, generating a curve cluster according to the frequency spectrum, and in response to determining a curve that meets a preset condition in the curve cluster, acquiring a jumped heart rate according to the curve that meets the preset condition to track the heart rate. In this way, by a mechanism of heart rate jump, a correct heart rate may be tracked in time, thereby largely improving an accuracy of heart rate prediction.

## Description

### CROSS-REFERENCE TO RELATED APPLICATION

This application claims priority to and benefits of Chinese Patent Application No. 202110455923.6, filed with the China National Intellectual Property Administration on April 26, 2021, the entire content of which is incorporated herein by reference.

### FIELD

The present disclosure relates to a technical field of electronic devices, and more particularly to a wearable device, as well as a heart rate tracking method and a heart rate tracking apparatus thereof.

### BACKGROUND

With an increasing living standard, wearable devices are becoming more and more popular. A heart rate tracking algorithm based on a Photo Pethysmo Graphic (PPG) signal is an essential algorithm for wearable devices. The heart rate tracking algorithm generally searches for a current heart rate value near a heart rate at a previous moment, such as +-6BPM (Beat Per Minute). However, after removing interferences, if the quality of the PPG signal is not good, the predicted heart rate value will be brought into a wrong range, which cannot obtain a real heart rate value.

Therefore, it is necessary to improve an accuracy of heart rate tracking of wearable devices.

### SUMMARY

Embodiments of the present disclosure seek to solve at least one of the problems existing in the related art to at least some extent.

According to embodiments of the present disclosure, there is provided a heart rate tracking method for a wearable device, including: obtaining a photo pethysmo graphic (PPG) signal of the wearable device within a preset time; obtaining a frequency spectrum corresponding to the PPG signal by a short-time Fourier transform of the PPG signal; generating a curve cluster according to the frequency spectrum; and in response to determining a curve that meets a preset condition in the curve cluster, acquiring a jumped heart rate according to the curve that meets the preset condition to track the heart rate.

According to the heart rate tracking method for the wearable device in some embodiments of the present disclosure, the PPG signal of the wearable device is obtained within the preset time, and the frequency spectrum corresponding to the PPG signal is obtained by the short-time Fourier transform of the PPG signal. Then, the curve cluster is generated according to the frequency spectrum, and the curve that meets the preset condition in the curve cluster is determined. When the curve that meets the preset condition exists, the jumped heart rate is acquired according to the curve that meets the preset condition to track the heart rate. In this way, the method provides a mechanism of heart rate jump, which may track a correct heart rate in time, thereby largely improving an accuracy of heart rate prediction.

In some embodiments, generating the curve cluster according to the frequency spectrum includes: performing amplitude normalization on the frequency spectrum; obtaining a point pair of frequency and amplitude by searching for a local peak for the normalized frequency spectrum; in response to determining a current moment greater than zero, pairing a point pair at the current moment with a point pair at a previous moment; and generating the curve cluster according to the two paired point pairs after pairing is successful.

In some embodiments, generating the curve cluster according to the two paired point pairs includes: in response to determining the point pair at the previous moment existing in a first curve, adding the point pair at the current moment to the first curve; or in response to determining that the point pair at the previous moment is not in the first curve, generating a second curve according to the point pair at the previous moment and the point pair at the current moment; and generating the curve cluster by acquiring the first curve and the second curve within the preset time.

In some embodiments, after obtaining the point pair of frequency and amplitude by searching for the local peak for the normalized frequency spectrum, the method further includes: in response to determining an amplitude of the point pair smaller than a preset amplitude, deleting the point pair whose amplitude is smaller than the preset amplitude.

In some embodiments, determining the curve that meets the preset condition in the curve cluster includes: selecting from all curves of the curve cluster a curve where a point pair at a last moment is consistent with the point pair at the current moment to generate a curve set; and in response to determining the curve set being not empty, selecting a curve that meets the preset condition from the curve set. The preset condition is that at least one curve in the curve set has a length larger than a preset length, and no other curves exist in a preset frequency range of the at least one curve within a preset period before the current moment.

In some embodiments, in response to determining the curve that meets the preset condition in the curve cluster, acquiring the jumped heart rate according to the curve that meets the preset condition to track the heart rate, includes: selecting from the curves that meet the preset condition a curve with a largest length, and determining a frequency at a last moment of the curve with the largest length as the jumped heart rate to track the heart rate.

In some embodiments, before obtaining the PPG signal of the wearable device, the method further includes: collecting an optical signal through an optical sensor, in which the optical signal includes the PPG signal and an acceleration signal; and filtering out the acceleration signal from the optical signal by a filtering algorithm to obtain the PPG signal.

According to embodiments of the present disclosure, there is provided a heart rate tracking apparatus for a wearable device, including: an obtaining module, configured to obtain a PPG signal of the wearable device within a preset time; a processing module, configured to obtain a frequency spectrum corresponding to the PPG signal by a short-time Fourier transform of the PPG signal; a generating module, configured to generate a curve cluster according to the frequency spectrum; and a tracking module, configured to acquire a jumped heart rate according to a curve that meets a preset condition to track the heart rate when it is determined that the curve that meets the preset condition exists in the curve cluster.

According to the heart rate tracking apparatus for the wearable device in some embodiments of the present disclosure, the PPG signal of the wearable device is obtained by the obtaining module within the preset time, and the frequency spectrum corresponding to the PPG signal is obtained by the processing module through the short-time Fourier transform of the PPG signal. Then, the curve cluster is generated by the generating module according to the frequency spectrum, and the curve that meets a preset condition in the curve cluster is determined by the tracking module. When the curve that meets the preset condition exists, the jumped heart rate is acquired according to the curve that meets the preset condition to track the heart rate. In this way, the apparatus is used based on a mechanism of heart rate jump, which may track a correct heart rate in time, thereby largely improving an accuracy of heart rate prediction.

In some embodiments, the generating module includes: a processing unit, configured to perform amplitude normalization on the frequency spectrum; a searching unit, configured to obtain a point pair of frequency and amplitude by searching for a local peak for the normalized frequency spectrum; a pairing unit, configured to pair a point pair at a current moment with a point pair at a previous moment when it is determined that the current moment is greater than zero; and a first generating unit, configured to generate the curve cluster according to the two paired point pairs after pairing is successful.

In some embodiments, the first generating unit is configured to: in response to determining the point pair at the previous moment existing in a first curve, add the point pair at the current moment to the first curve; or in response to determining that the point pair at the previous moment is not in the first curve, generate a second curve according to the point pair at the previous moment and the point pair at the current moment; and generate the curve cluster by acquiring the first curve and the second curve within the preset time.

In some embodiments, the generating module further includes: a deleting unit, configured to delete a point pair whose amplitude is smaller than a preset amplitude when it is determined that an amplitude of the point pair is smaller than the preset amplitude.

In some embodiments, the judging module includes: a second generating unit, configured to select from all curves of the curve cluster a curve where a point pair at a last moment is consistent with the point pair at the current moment to generate a curve set; and a selecting unit, configured to select a curve that meets the preset condition from the curve set when it is determined that the curve set is not empty. The preset condition is that at least one curve in the curve set has a length larger than a preset length, and no other curves exist in a preset frequency range of the at least one curve within a preset period before the current moment.

In some embodiments, the tracking module is configured to: select from the curves that meet the preset condition a curve with a largest length, and determine a frequency at a last moment of the curve with the largest length as the jumped heart rate to track the heart rate.

In some embodiments, the heart rate tracking apparatus for the wearable device further includes: a collecting module, configured to collect an optical signal through an optical sensor, in which the optical signal includes the PPG signal and an acceleration signal; and a filtering module, configured to filter out the acceleration signal from the optical signal by a filtering algorithm to obtain the PPG signal.

According to embodiments of the present disclosure, there is provided a wearable device, including the above-mentioned heart rate tracking apparatus for the wearable device.

According to the wearable device in some embodiments of the present disclosure, the heart rate tracking apparatus of the wearable device is used based on a mechanism of heart rate jump, which may track a correct heart rate in time, thereby largely improving an accuracy of heart rate prediction.

According to embodiments of the present disclosure, there is provided an electronic device, including a processor and a memory for storing instructions executable by the processor. The processor is configured to execute the instructions to perform the above-mentioned heart rate tracking method for the wearable device.

According to the electronic device in some embodiments of the present disclosure, the above-mentioned heart rate tracking method for the wearable device is performed based on a mechanism of heart rate jump, which may track a correct heart rate in time, thereby largely improving an accuracy of heart rate prediction.

According to embodiments of the present disclosure, there is provided a non-transitory computer-readable storage medium, having stored therein instructions that, when executed by a processor of an electronic device, causes the electronic device to perform the above-mentioned heart rate tracking method for the wearable device.

According to the non-transitory computer-readable storage medium in some embodiments of the present disclosure, the above-mentioned heart rate tracking method for the wearable device is performed based on a mechanism of heart rate jump, which may track a correct heart rate in time, thereby largely improving an accuracy of heart rate prediction.

### BRIEF DESCRIPTION OF THE DRAWINGS

FIG. 1 is a flow chart of a heart rate tracking method for a wearable device in some embodiments of the present disclosure;
FIG. 2 is a schematic diagram showing a real heart rate and a curve cluster in an embodiment of the present disclosure;
FIG. 3 is a schematic diagram showing a predicted heart rate obtained by a heart rate tracking method for a wearable device in a related art;
FIG. 4 is a schematic diagram showing a predicted heart rate obtained by a heart rate tracking method for a wearable device in some embodiments of the present disclosure;
FIG. 5 is a schematic diagram showing a generating process of a curve cluster in a heart rate tracking method for a wearable device in some embodiments of the present disclosure;
FIG. 6 is a block diagram of a heart rate tracking apparatus for a wearable device in some embodiments of the present disclosure;
FIG. 7 is a block diagram of a wearable device in some embodiments of the present disclosure;
FIG. 8 is a block diagram of an electronic device in some embodiments of the present disclosure.

### DETAILED DESCRIPTION

Embodiments of the present disclosure are described in detail below, examples of which are illustrated in the drawings. The same or similar elements are denoted by the same or similar reference numerals in different drawings, unless indicated otherwise. The embodiments described herein with reference to drawings are explanatory, and used to generally understand the present disclosure. The embodiments shall not be construed to limit the present disclosure.

A heart rate tracking method for a wearable device, a heart rate tracking apparatus for a wearable device, a wearable device, an electronic device and a non-transitory computer-readable storage medium in some embodiments of the present disclosure will be described below with reference to drawings.

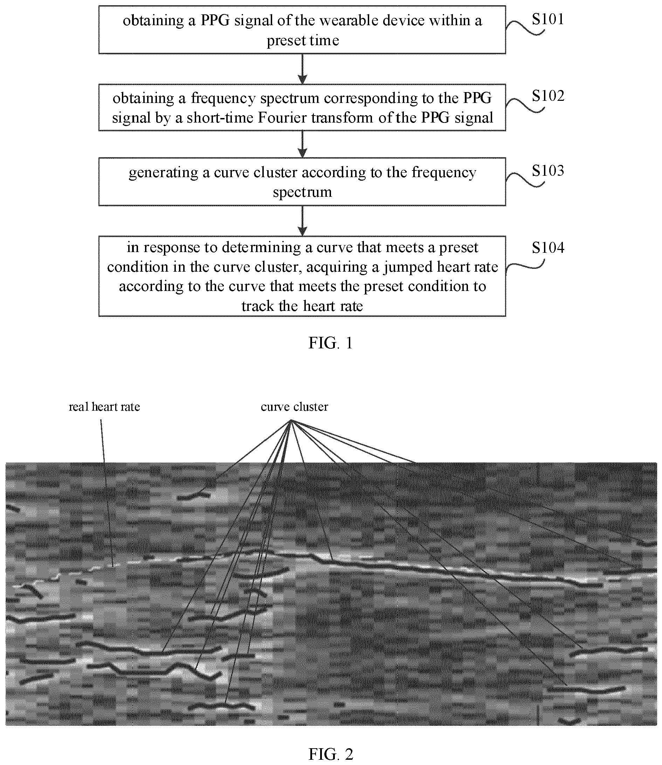

FIG. 1 is a flow chart of a heart rate tracking method for a wearable device in some embodiments of the present disclosure.

In some embodiments of the present disclosure, the wearable device may be a smart watch or a smart bracelet.

As shown in FIG. 1, the heart rate tracking method for the wearable device in some embodiments of the present disclosure includes steps S101 to S104.

In step S101, a photo pethysmo graphic (PPG) signal of the wearable device is obtained within a preset time.

The preset time may be set according to actual needs, such as 8s. The photo pethysmo graphic is a method where light is irradiated into a skin and light scattering caused by blood flow is measured. Specifically, green light emitted by a light emitting diode (LED) in an optical sensor passes through tissues, arteries and veins in the skin, and is absorbed or reflected back to the optical sensor. The green light reflected back to the optical sensor has a certain attenuation. Unlike blood, the absorption of the green light by muscles, bones, veins and other connecting tissues is basically unchanged (provided that there is no significant movement of measured sites). The blood flows in the arteries, such that the absorption of the green light by the arteries changes. The green light is then transformed into an electronic signal, and the electronic signal may be divided into a direct current (DC) signal and an alternating current (AC) signal due to the changeable absorption of the green light by the arteries and the basically unchangeable absorption of the green light by other tissues. Finally, characteristics of blood flow may be reflected by extracting the AC signal.

In some embodiments, before obtaining the PPG signal of the wearable device, the method further includes collecting an optical signal through an optical sensor, in which the optical signal includes the PPG signal and an acceleration signal; and filtering out the acceleration signal from the optical signal by a filtering algorithm to obtain the PPG signal.

In this step, the PPG signal and the acceleration signal may be collected by the optical sensor, such as a PPG optical module. A motion signal, such as the acceleration signal may be filtered out from the PPG signal by the relevant filtering algorithm, such as recursive least squares (PLS), and then the filtered PPG signal is stored for a certain time, such as the preset time of 8s.

In step S102, a frequency spectrum corresponding to the PPG signal is obtained by a short-time Fourier transform of the PPG signal.

In this step, the short-time Fourier transform is performed on a certain amount of the PPG signal to obtain an amplitude value in a certain frequency spectrum interval at a moment of 8s.

In step S103, a curve cluster is generated according to the frequency spectrum.

In some embodiments, generating the curve cluster according to the frequency spectrum includes performing amplitude normalization on the frequency spectrum, obtaining a point pair of frequency and amplitude by searching for a local peak for the normalized frequency spectrum, in response to determining a current moment greater than zero, pairing a point pair at the current moment with a point pair at a previous moment, and generating the curve cluster according to the two paired point pairs after pairing is successful.

In some embodiments, generating the curve cluster according to the two paired point pairs includes in response to determining the point pair at the previous moment existing in a first curve, adding the point pair at the current moment to the first curve; or in response to determining that the point pair at the previous moment is not in the first curve, generating a second curve according to the point pair at the previous moment and the point pair at the current moment; and generating the curve cluster by acquiring the first curve and the second curve within the preset time.

In this step, normalization is performed on a maximum value and a minimum value of an amplitude of the frequency spectrum to obtain the normalized amplitude between 0 and 1. For example, normalization may be performed by dividing the amplitude by a maximum amplitude. Then, the point pair of frequency and amplitude may be obtained by searching for the local peak for the normalized frequency spectrum, such that many point pairs may be obtained.

If the current moment is zero moment, no processing is performed. If the current moment is a moment greater than zero moment, a point pair at the current moment and a point pair at a previous moment are paired according to a pairing condition. In an implementation, the pairing condition is that the difference between a frequency of the point pair at the current moment and a frequency of the point pair at the previous moment is within 6 BPM. If the point pair at the current moment and the point pair at the previous moment meet the pairing condition, it is determined whether the point pair at the previous moment exists in a first curve. In response to determining the point pair at the previous moment existing in the first curve, the point pair at the current moment is added to the first curve. That is to say, a length of the first curve is increased. In response to determining that the point pair at the previous moment does not exist in the first curve, a new curve is generated as a second curve according to the point pair at the previous moment and the point pair at the current moment. All the generated curves including the first curve and the second curve within the preset time form the curve cluster.

With the increase of time, steps S101 to S103 are performed cyclically to obtain continuously growing curve clusters, as shown in FIG. 2. It should be noted that only some of the curves are marked in the curve cluster in FIG. 2, and the curves with the same thickness are also curves in the curve cluster.

In order to further improve the accuracy of heart rate prediction, after obtaining the point pair of frequency and amplitude by searching for the local peak for the normalized frequency spectrum, the method further includes in response to determining an amplitude of the point pair smaller than a preset amplitude, deleting the point pair whose amplitude is smaller than the preset amplitude. The preset amplitude may be set according to actual needs, such as 0.6.

That is to say, in order to further improve the accuracy of heart rate prediction, after obtaining the point pair of frequency and amplitude by searching for the local peak for the frequency spectrum, for the normalized amplitude, it is necessary to determine whether an amplitude of the point pair is smaller than a preset amplitude, and to delete the point pair whose amplitude is smaller than the preset amplitude, such as 0.6.

In step S104, in response to determining a curve that meets a preset condition in the curve cluster, a jumped heart rate is acquired according to the curve that meets the preset condition to track the heart rate.

In some embodiments, determining the curve that meets the preset condition in the curve cluster includes selecting from all curves of the curve cluster a curve where a point pair at a last moment is consistent with the point pair at the current moment to generate a curve set; and in response to determining the curve set being not empty, selecting a curve that meets the preset condition from the curve set. The preset condition is that at least one curve in the curve set has a length larger than a preset length, and no other curves exist in a preset frequency range of the at least one curve within a preset period before the current moment. The preset length, the preset frequency range and the preset moment may be set according to actual needs. For example, the preset length may be 5, the preset frequency range may be in a range of 0 to 0.5 Hz, and the preset period may be a period of 5 moments before the current moment.

In some embodiments, in response to determining the curve that meets the preset condition in the curve cluster, acquiring the jumped heart rate according to the curve that meets the preset condition to track the heart rate, includes selecting from the curves that meet the preset condition a curve with a largest length, and determining a frequency at a last moment of the curve with the largest length as the jumped heart rate to track the heart rate.

A heart rate tracking algorithm is generally performed by the steps as follows.

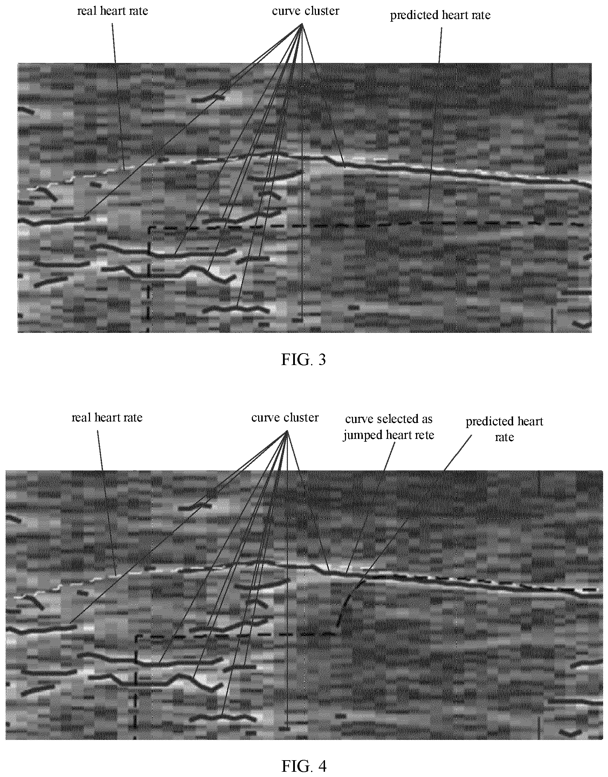

A heart rate at a previous moment is first obtained to search for a new heart rate at a current moment before or after 6 BPM of the heart rate at the previous moment. When the heart rate signal is weak, a heart rate tracking error will be amplified, and when the heart rate signal is strong, the algorithm cannot track the heart rate correctly, for example, as shown in FIG. 3.

For this, the present disclosure provides an improved algorithm as follows.

After obtaining a curve cluster, a curve where a point pair at a last moment is consistent with the point pair at the current moment is selected from all curves of the curve cluster to generate a curve set. Then, it is determined whether the curve set is empty. In response to determining the curve set being not empty, there is a curve L with a length larger than 5, and with no other curves existing in a 0.5Hz range of the curve L within a period of 5 moments before the current moment. When there are more than one curve L, a curve with a largest length is selected, and a frequency at a last moment of the curve with the largest length is determined as the current heart rate to be jumped to so as to track the heart rate, as shown in FIG. 4.

In this way, the method provides a mechanism of heart rate jump, which may track a correct heart rate in time, thereby largely improving an accuracy of heart rate prediction.

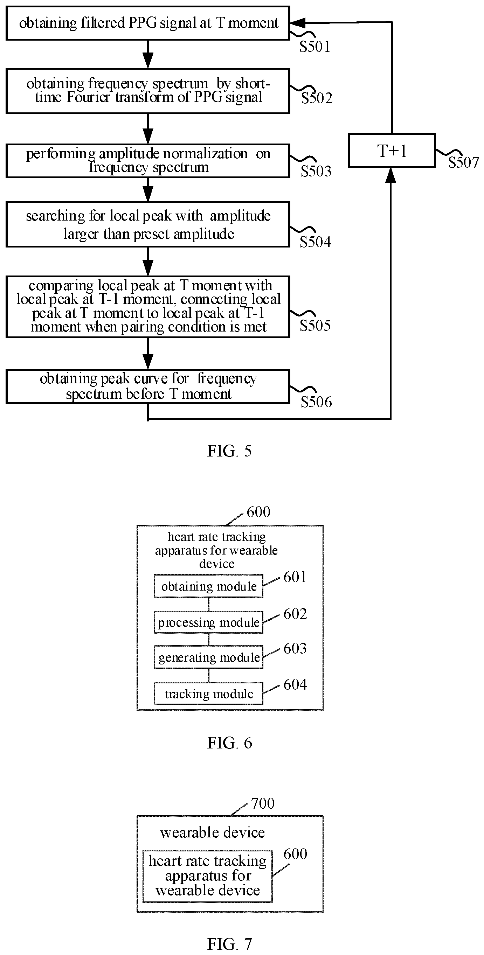

In order to enable those skilled in the art to further understand steps S101 to S103, FIG. 5 is a schematic diagram showing a generating process of a curve cluster in a heart rate tracking method for a wearable device in some embodiments of the present disclosure. As shown in FIG. 5, the generating process of the curve cluster in the heart rate tracking method for the wearable device includes steps of S501 to S507.

In step S501, a filtered PPG signal is obtained at T moment.

In step S502, a frequency spectrum corresponding to the PPG signal is obtained by a short-time Fourier transform.

In step S503, amplitude normalization is performed on the frequency spectrum. For example, normalization may be performed by dividing an amplitude by a maximum amplitude to obtain the normalized amplitude between 0 and 1.

In step S504, a local peak with an amplitude larger than a preset amplitude is obtained. The preset amplitude may be 0.6.

In step S505, a local peak at T moment is compared with a local peak at T-1 moment, and connected to the local peak at T-1 moment when a pairing condition is met. In an implementation, the pairing condition is that the difference between a frequency of the point pair at the current moment and a frequency of the point pair at the previous moment is within 6 BPM.

In step S506, a peak curve for the frequency spectrum before T moment is obtained.

In step S507, a filtered PPG signal at T+1 moment is processed continually.

After obtaining the curve cluster by steps S501 to S507, a curve where a point pair at a last moment is consistent with the point pair at the current moment is selected from all curves of the curve cluster to generate a curve set. Then, it is determined whether the curve set is empty. In response to determining the curve set being not empty, there is a curve L with a length larger than 5, and with no other curves existing in a 0.5Hz range of the curve L within a period of 5 moments before the current moment. When there is more than one curve L, a curve with a largest length is selected, and a frequency at a last moment of the curve with the largest length is determined as the current heart rate to be jumped to so as to track the heart rate, as shown in FIG. 4.

According to the heart rate tracking method for the wearable device in some embodiments of the present disclosure, the PPG signal of the wearable device is obtained within the preset time, and the frequency spectrum corresponding to the PPG signal is obtained by the short-time Fourier transform of the PPG signal. Then, the curve cluster is generated according to the frequency spectrum, and the curve that meets a preset condition in the curve cluster is determined. When the curve that meets the preset condition exists, the jumped heart rate is acquired according to the curve that meets the preset condition to track the heart rate. In this way, the method provides a mechanism of heart rate jump, which may track a correct heart rate in time, thereby largely improving an accuracy of heart rate prediction.

FIG. 6 is a block diagram of a heart rate tracking apparatus for a wearable device in some embodiments of the present disclosure.

As shown in FIG. 6, the heart rate tracking apparatus 600 for the wearable device in some embodiments of the present disclosure includes an obtaining module 601, a processing module 602, a generating module 603 and a tracking module 604.

The obtaining module 601 is configured to obtain a PPG signal of the wearable device within a preset time. The processing module 602 is configured to obtain a frequency spectrum corresponding to the PPG signal by a short-time Fourier transform of the PPG signal. The generating module 603 is configured to generate a curve cluster according to the frequency spectrum. The tracking module 604 is configured to acquire a jumped heart rate according to a curve that meets a preset condition to track the heart rate when it is determined that the curve that meets the preset condition exists in the curve cluster.

In some embodiments, the generating module 603 includes a processing unit, a searching unit, a pairing unit and a first generating unit. The processing unit is configured to perform amplitude normalization on the frequency spectrum. The searching unit is configured to obtain a point pair of frequency and amplitude by searching for a local peak for the normalized frequency spectrum. The pairing unit is configured to pair a point pair at a current moment with a point pair at a previous moment when it is determined that the current moment is greater than zero. The first generating unit is configured to generate the curve cluster according to the two paired point pairs after pairing is successful.

In some embodiments, the first generating unit is configured to in response to determining the point pair at the previous moment existing in a first curve, add the point pair at the current moment to the first curve; or in response to determining that the point pair at the previous moment is not in the first curve, generate a second curve according to the point pair at the previous moment and the point pair at the current moment; generate the curve cluster by acquiring the first curve and the second curve within the preset time.

In some embodiments, the generating module 603 further includes a deleting unit. The deleting unit is configured to delete a point pair whose amplitude is smaller than a preset amplitude when it is determined that an amplitude of the point pair is smaller than the preset amplitude.

In some embodiments, the judging module 604 includes a second generating unit and a selecting unit. The second generating unit is configured to select from all curves of the curve cluster a curve where a point pair at a last moment is consistent with the point pair at the current moment to generate a curve set. The selecting unit is configured to select a curve that meets the preset condition from the curve set when it is determined that the curve set is not empty. The preset condition is that at least one curve in the curve set has a length larger than a preset length, and no other curves exist in a preset frequency range of the at least one curve within a preset period before the current moment.

In some embodiments, the tracking module 605 is configured to select from the curves that meet the preset condition a curve with a largest length, and determine a frequency at a last moment of the curve with the largest length as the jumped heart rate to track the heart rate.

In some embodiments, the heart rate tracking apparatus 600 for the wearable device further includes a collecting module and a filtering module. The collecting module is configured to collect an optical signal through an optical sensor, in which the optical signal includes the PPG signal and an acceleration signal. The filtering module is configured to filter out the acceleration signal from the optical signal by a filtering algorithm to obtain the PPG signal.

It should be noted that, for details not disclosed in the heart rate tracking apparatus for the wearable device in the embodiments of the present disclosure, reference is made to the details disclosed in the heart rate tracking method for the wearable device in the embodiments of the present disclosure, which will not be described in detail here.

According to the heart rate tracking apparatus for the wearable device in some embodiments of the present disclosure, the PPG signal of the wearable device is obtained by the obtaining module within the preset time, and the frequency spectrum corresponding to the PPG signal is obtained by the processing module through the short-time Fourier transform of the PPG signal. Then, the curve cluster is generated by the generating module according to the frequency spectrum, and the curve that meets a preset condition in the curve cluster is determined by the tracking module. When the curve that meets the preset condition exists, the jumped heart rate is acquired according to the curve that meets the preset condition to track the heart rate. In this way, the apparatus is used based on a mechanism of heart rate jump, which may track a correct heart rate in time, thereby largely improving an accuracy of heart rate prediction.

FIG. 7 is a block diagram of a wearable device in some embodiments of the present disclosure.

As shown in FIG. 7, the wearable device 700 provided in some embodiments of the present disclosure includes the above-mentioned heart rate tracking apparatus 600 for the wearable device.

By the above-mentioned heart rate tracking apparatus for the wearable device, the wearable device provided in some embodiments of the present disclosure may be used based on a mechanism of heart rate jump, which may track a correct heart rate in time, thereby largely improving an accuracy of heart rate prediction.

According to the above-mentioned embodiments, an electronic device is provided, including a processor and a memory for storing instructions executable by the processor. The processor is configured to execute the instructions to perform the above-mentioned heart rate tracking method for the wearable device.

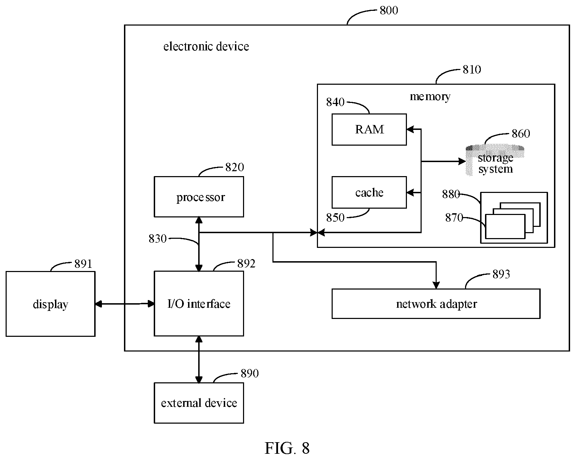

FIG. 8 is a block diagram of an electronic device in some embodiments of the present disclosure.

As shown in FIG. 8, the electronic device 800 includes a memory 810, a processor 820 and a bus 830 that couples various components, including the memory 810 and the processor 820.

The memory 810 is configured to store instructions executable by the processor 820. The processor 801 is configured to call and execute the instructions stored in the memory 802 to perform the above-mentioned heart rate tracking method for the wearable device.

The bus 830 represents one or more of any of several types of bus architectures, including a memory bus or a memory controller, a peripheral bus, an accelerated graphics port, and a processor or a local bus using any of a variety of bus architectures. By way of example, such architectures include but are not limited to an Industry Standard Architecture (ISA) bus, a Micro Channel Architecture (MCA) bus, an Enhanced ISA (EISA) bus, a Video Electronics Standards Association (VESA) local bus, and a Peripheral Component Interconnects (PCI) bus.

The electronic device 800 typically includes a variety of electronic device readable media. Such media may be any available media that are accessible by the electronic device 800, and include both volatile and non-volatile media, as well as removable and non-removable media.

The memory 810 may include computer system readable media in the form of volatile memory, such as a random access memory (RAM) 840 and/or a cache memory 850. The electronic device 800 may further include other removable/non-removable, volatile/non-volatile computer system storage media. By way of example only, the storage system 860 may be provided for reading from or writing to non-removable, non-volatile magnetic media (not shown in FIG. 8 and typically called a "hard drive"). Although not shown in FIG. 8, a magnetic disk drive for reading from or writing to a removable, non-volatile magnetic disk (e.g., a "floppy disk"), and an optical disk drive for reading from or writing to a removable, non-volatile optical disk such as a CD-ROM, DVD-ROM, or other optical media can be provided. In such instances, each drive can be connected to the bus 830 by one or more data media interfaces. The memory 810 may include at least one program product having a set (e.g., at least one) of program modules that are configured to carry out the functions of embodiments of the present disclosure.

For example, a program/utility 880, having a set (at least one) of program modules 870, may be stored in the memory 810. Such program modules 870 include but are not limited to an operating system, one or more functions, other program modules, and program data. Each of the operating system, one or more functions, other program modules, and program data or some combination thereof, may include an implementation of a networking environment. The program modules 870 are generally configured to carry out the functions and/or methods of embodiments of the present disclosure as described herein.

The electronic device 800 may also communicate with one or more external devices 890 such as a keyboard, a pointing device, a display 891, etc.; one or more devices that enable a consumer to interact with the electronic device 800; and/or any devices (e.g., network cards, modems, etc.) that enable the electronic device 800 to communicate with one or more other computing devices. Such communication can occur via I/O interfaces 892. In addition, the electronic device 800 can communicate with one or more networks such as a local area network (LAN), a wide area network (WAN), and/or a public network (e.g., the Internet) via a network adapter 893. As depicted, the network adapter 893 communicates with other components of the electronic device 800 via the bus 830. It should be understood that although not shown, other hardware and/or software components could be used in conjunction with the electronic device 800, including, but not limited to: microcodes, device drivers, redundant processing units, external disk drive arrays, RAID systems, tape drives, and data backup storage systems, etc.

The processor 820 is configured to execute the instructions stored in the memory 810 to perform various functions and process data.

It should be noted that concerning an implementation process of the electronic device in some embodiments of the present disclosure, reference may be made to the above-mentioned method in the embodiments of the present disclosure, which will not be repeated here in detail.

The electronic device provided in some embodiments of the present disclosure may be configured to perform the above-mentioned heart rate tracking method for the wearable device based on a mechanism of heart rate jump, which may track a correct heart rate in time, thereby largely improving an accuracy of heart rate prediction.

According to the above-mentioned embodiments, a non-transitory computer-readable storage medium is provided, having stored therein instructions that, when executed by a processor of an electronic device, causes the electronic device to perform the above-mentioned heart rate tracking method for the wearable device.

The non-transitory computer-readable storage medium provided in some embodiments of the present disclosure may be configured to perform the above-mentioned heart rate tracking method for the wearable device based on a mechanism of heart rate jump, which may track a correct heart rate in time, thereby largely improving an accuracy of heart rate prediction.

According to the above-mentioned embodiments, a computer program product is provided, which, when executed by a processor of an electronic device, causes the electronic device to perform the above-mentioned heart rate tracking method for the wearable device.

The computer program product provided in some embodiments of the present disclosure may be configured to perform the above-mentioned heart rate tracking method for the wearable device by a mechanism of heart rate jump, which may track a correct heart rate in time, thereby largely improving an accuracy of heart rate prediction.

In the specification, it is to be understood that terms such as "central", "longitudinal", "lateral", "length", "width", "thickness", "upper", "lower", "front", "rear", "left", "right", "vertical", "horizontal", "top", "bottom", "inner", "outer", "clockwise", "counterclockwise", "axial", "radial" and "circumferential" should be construed to refer to the orientation or positional relationship as shown in the drawings. These relative terms are for convenience of description and do not require that the present disclosure be constructed or operated in a particular orientation, which shall not be construed to limit the present disclosure.

In addition, terms such as "first" and "second" are used herein for purposes of description and are not intended to indicate or imply relative importance or significance or to imply the number of indicated technical features. Thus, the feature defined with "first" or "second" may comprise one or more of this feature. In the description of the present disclosure, "a plurality of' means two or more, unless specified otherwise.

In the present disclosure, unless specified or limited otherwise, the terms "mounted," "connected," "coupled," "fixed" and the like are used broadly, and may be, for example, fixed connections, detachable connections, or integral connections; may also be mechanical or electrical connections; may also be direct connections or indirect connections via intervening structures; may also be inner communications or interactions of two elements, which can be understood by those skilled in the art according to specific situations.

In the specification of the present disclosure, unless specified or limited otherwise, a structure in which a first feature is "on" or "below" a second feature may include an embodiment in which the first feature is in direct contact with the second feature, and may also include an embodiment in which the first feature and the second feature are not in direct contact with each other, but are contacted via an additional feature formed therebetween. Furthermore, a first feature "on," "above," or "on top of' a second feature may include an embodiment in which the first feature is right or obliquely "on," "above," or "on top of' the second feature, or just means that the first feature is at a height higher than that of the second feature; while a first feature "below," "under," or "on bottom of' a second feature may include an embodiment in which the first feature is right or obliquely "below," "under," or "on bottom of' the second feature, or just means that the first feature is at a height lower than that of the second feature.

Reference throughout this specification to "an embodiment," "some embodiments," "an example," "a specific example," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example," "in an example," "in a specific example," or "in some examples," in various places throughout this specification are not necessarily referring to the same embodiment or example of the present disclosure. Furthermore, the particular features, structures, materials, or characteristics may be combined in any suitable manner in one or more embodiments or examples. In addition, the features described in some embodiments or examples may be combined or connected with the features of other embodiments or examples by those skilled in the art without contradicting with each other.

Although explanatory embodiments have been shown and described, it would be appreciated by those skilled in the art that the above embodiments cannot be construed to limit the present disclosure, and changes, alternatives, and modifications can be made in the embodiments without departing from the scope of the present disclosure.

## Claims

1. A heart rate tracking method for a wearable device, comprising:
obtaining a photo pethysmo graphic (PPG) signal of the wearable device within a preset time;
obtaining a frequency spectrum corresponding to the PPG signal by a short-time Fourier transform of the PPG signal;
generating a curve cluster according to the frequency spectrum; and
in response to determining a curve that meets a preset condition in the curve cluster, acquiring a jumped heart rate according to the curve that meets the preset condition to track the heart rate.

2. The method according to claim 1, wherein generating the curve cluster according to the frequency spectrum comprises:
performing amplitude normalization on the frequency spectrum;
obtaining a point pair of frequency and amplitude by searching for a local peak for the normalized frequency spectrum;
in response to determining a current moment greater than zero, pairing a point pair at the current moment with a point pair at a previous moment; and
generating the curve cluster according to the two paired point pairs after pairing is successful.

3. The method according to claim 2, wherein generating the curve cluster according to the two paired point pairs comprises:
in response to determining the point pair at the previous moment existing in a first curve, adding the point pair at the current moment to the first curve; or
in response to determining that the point pair at the previous moment is not in the first curve, generating a second curve according to the point pair at the previous moment and the point pair at the current moment; and
generating the curve cluster by acquiring the first curve and the second curve within the preset time.

4. The method according to claim 2 or 3, after obtaining the point pair of frequency and amplitude by searching for the local peak for the normalized frequency spectrum, further comprising:
in response to determining an amplitude of the point pair smaller than a preset amplitude, deleting the point pair whose amplitude is smaller than the preset amplitude.

5. The method according to any one of claims 1 to 4, wherein determining the curve that meets the preset condition in the curve cluster comprises:
selecting from all curves of the curve cluster a curve where a point pair at a last moment is consistent with a point pair at a current moment to generate a curve set; and
in response to determining the curve set being not empty, selecting a curve that meets the preset condition from the curve set;
wherein the preset condition is that at least one curve in the curve set has a length larger than a preset length, and no other curves exist in a preset frequency range of the at least one curve within a preset period before the current moment.

6. The method according to claim 5, wherein in response to determining the curve that meets the preset condition in the curve cluster, acquiring the jumped heart rate according to the curve that meets the preset condition to track the heart rate, comprises:
selecting from the curves that meet the preset condition a curve with a largest length, and determining a frequency at a last moment of the curve with the largest length as the jumped heart rate to track the heart rate.

7. The method according to any one of claims 1 to 6, before obtaining the PPG signal of the wearable device, further comprising:
collecting an optical signal through an optical sensor, wherein the optical signal comprises the PPG signal and an acceleration signal; and
filtering out the acceleration signal from the optical signal by a filtering algorithm to obtain the PPG signal.

8. A heart rate tracking apparatus for a wearable device, comprising:
an obtaining module, configured to obtain a PPG signal of the wearable device within a preset time;
a processing module, configured to obtain a frequency spectrum corresponding to the PPG signal by a short-time Fourier transform of the PPG signal;
a generating module, configured to generate a curve cluster according to the frequency spectrum; and
a tracking module, configured to acquire a jumped heart rate according to a curve that meets a preset condition to track the heart rate when it is determined that the curve that meets the preset condition exists in the curve cluster.

9. The apparatus according to claim 8, wherein the generating module comprises:
a processing unit, configured to perform amplitude normalization on the frequency spectrum;
a searching unit, configured to obtain a point pair of frequency and amplitude by searching for a local peak for the normalized frequency spectrum;
a pairing unit, configured to pair a point pair at a current moment with a point pair at a previous moment when it is determined that the current moment is greater than zero; and
a first generating unit, configured to generate the curve cluster according to the two paired point pairs after pairing is successful.

10. The apparatus according to claim 9, wherein the first generating unit is configured to:
in response to determining the point pair at the previous moment existing in a first curve, add the point pair at the current moment to the first curve; or
in response to determining that the point pair at the previous moment is not in the first curve, generate a second curve according to the point pair at the previous moment and the point pair at the current moment; and
generate the curve cluster by acquiring the first curve and the second curve within the preset time.

11. The apparatus according to claim 9 or 10, wherein the generating module further comprises:
a deleting unit, configured to delete a point pair whose amplitude is smaller than a preset amplitude when it is determined that an amplitude of the point pair is smaller than the preset amplitude.

12. The apparatus according to any one of claims 8 to 11, wherein the judging module comprises:
a second generating unit, configured to select from all curves of the curve cluster a curve where a point pair at a last moment is consistent with the point pair at the current moment to generate a curve set; and
a selecting unit, configured to select a curve that meets the preset condition from the curve set when it is determined that the curve set is not empty;
wherein the preset condition is that at least one curve in the curve set has a length larger than a preset length, and no other curves exist in a preset frequency range of the at least one curve within a preset period before the current moment.

13. The apparatus according to claim 12, wherein the tracking module is configured to:
select from the curves that meet the preset condition a curve with a largest length, and determine a frequency at a last moment of the curve with the largest length as the jumped heart rate to track the heart rate.

14. The apparatus according to any one of claims 8 to 13, further comprising:
a collecting module, configured to collect an optical signal through an optical sensor, wherein the optical signal comprises the PPG signal and an acceleration signal; and
a filtering module, configured to filter out the acceleration signal from the optical signal by a filtering algorithm to obtain the PPG signal.

15. A wearable device, comprising the heart rate tracking apparatus according to any one of claims 8 to 14.

16. An electronic device, comprising:
a processor; and
a memory for storing instructions executable by the processor;
wherein the processor is configured to execute the instructions to perform the method according to any one of claims 1 to 7.

17. A non-transitory computer-readable storage medium, having stored therein instructions that, when executed by a processor of an electronic device, causes the electronic device to perform the method according to any one of claims 1 to 7.
